# FASCICULE DE BREVET EUROPEEN

(11) **EP 0 639 549 B1**
(45) Date de publication et mention de la délivrance du brevet: **07.01.1998**
(21) Numéro de dépôt: 94401784.7
(22) Date de dépôt: 03.08.1994
(51) Int. Cl.: C07C 6/04, B01J 23/36

(54) **Procédé de métathèse des oléfines mettant en oeuvre un catalyseur au rhénium amélioré**
Verfahren zur Metathese von Olefinen in dem ein verbesserter Rheniumkatalysator verwendet wird
Process for the metathesis of olefins using an improved rhenium catalyst

(30) Priorité: 20.08.1993 FR 9310196
(43) Date de publication de la demande: 22.02.1995
(73) Titulaire: INSTITUT FRANCAIS DU PETROLE, 92502 Rueil-Malmaison (FR)
(72) Inventeur: Chauvin Yves, 78230 Le Pecq (FR); Commereuc Dominique, 92190 Meudon (FR)
(74) Mandataire: Andreeff, François

(56) Documents cités:
- FR-A- 1 490 277
- FR-A- 1 516 853

## Description

La présente invention concerne un procédé de métathèse des oléfines utilisant un catalyseur amélioré à base de rhénium.

La métathèse des oléfines, ou réaction de redistribution des groupements alkylidènes entre eux, présente un grand intérêt pratique, par exemple pour le rééquilibrage entre elles des oléfines légères issues du craquage à la vapeur, telles que l'éthylène, le propylène et les butènes.

Différents types de catalyseurs sont susceptibles d'être mis en oeuvre dans la réaction de métathèse, soit homogènes, quand leurs éléments constitutifs sont tous solubles dans le milieu de la réaction, soit hétérogènes, quand au moins un des éléments est insoluble dans le dit milieu. Ces derniers sont particulièrement intéressants quand le métal actif est onéreux et qu'il est nécessaire d'envisager sa réutilisation sans pertes. C'est le cas des catalyseurs à base de rhénium, dont l'emploi sous forme hétérogène a été préconisé pour catalyser la métathèse des oléfines simples, par exemple dans les brevets US 3 642 931 et US 3 676 520.

Ces catalyseurs ont été préparés dans l'art antérieur en introduisant de l'oxyde de rhénium, par les méthodes habituelles de la catalyse hétérogène, sur des supports variés tels que décrits par exemple dans le brevet US 3 641 189. Parmi les divers supports, l'alumine, ou un support contenant de l'alumine, paraît présenter les propriétés les plus intéressantes pour conférer au catalyseur une bonne activité et une bonne stabilité.

De nombreuses modifications au catalyseur de base constitué par du rhénium sur alumine, ont été décrites pour améliorer ses propriétés. Il a ainsi été trouvé des effets bénéfiques à l'addition de composés alcalins ou alcalino-terreux (US 3 594 440, US 3 637 892), d'anions d'acides (US 3 697 613), d'oxydes d'étain (GB 1 377 161), d'oxyde de bore (US 5 055 628), ou d'éléments de terres rares (US 3 728 414).

L'invention décrit un procédé mettant en oeuvre des catalyseurs à base de rhénium plus actifs que ceux de l'art antérieur. Il a en effet été trouvé que l'addition au rhénium supporté sur alumine, ou sur un support contenant au moins 75 % en poids d'alumine, de composés du niobium et/ou du tantale améliore de façon inattendue l'activité de ces catalyseurs, permettant d'utiliser, pour une activité identique, des proportions plus faibles de rhénium, ce qui est important au vu du coût de ce métal.

Cet effet est d'autant plus surprenant que les solides analogues comportant des composés du niobium et/ou du tantale supportés sur alumine ou sur un support contenant au moins 75 % en poids d'alumine, mais exempts de rhénium, ne sont pas actifs comme catalyseurs de la réaction de métathèse.

Le catalyseur utilisé dans le procédé selon l'invention comprend donc au moins trois composants : un support poreux constitué par de l'alumine ou un composé contenant au moins 75 % en poids d'alumine, 0,01 à 30 % en poids de niobium et/ou de tantale sous forme oxyde, et 0,01 à 20 % en poids de rhénium sous forme oxyde.

Le support poreux est constitué par de l'alumine ou par un composé contenant au moins 75 % en poids d'alumine, qui doit avantageusement présenter une surface appréciable, par exemple au moins 10 m²/g, et de préférence au moins 50 m²/g, et un volume de pores suffisant, par exemple au moins 0,1 ml/g, et de préférence 0,3-1 ml/g. On peut utiliser avantageusement une alumine du même type que celles des catalyseurs de reformage catalytique. Les formes gamma cubique, éta, sont ainsi préférées.

Le composé du niobium et/ou du tantale est constitué par un halogénure ou un oxyhalogénure (par exemple le pentachlorure ou l'oxytrichlorure), par un alkoxyde (par exemple le pentaéthoxyde), ou de préférence par un oxyde hydraté remis en solution aqueuse par addition d'un complexant tel que par exemple l'acide oxalique et/ou les acides-alcools et notamment, l'acide tartrique, ou leurs sels d'ammonium. Le composé du niobium et/ou du tantale peut être introduit sur le support par toutes les méthodes habituelles de la catalyse hétérogène, par exemple par imprégnation en solution. On préfère généralement utiliser la méthode d'imprégnation à sec, dite aussi imprégnation capillaire. Le composé du niobium et/ou du tantale est mis en solution dans l'eau ou dans un solvant organique, par exemple un alcool. Le volume de la solution est inférieur ou au maximum égal au volume des pores du support. On règle la quantité de niobium et/ou de tantale sur le support par le choix de la concentration de la solution d'imprégnation. Lorsque la quantité que l'on désire imprégner est supérieure à celle que permet d'introduire une solution à sa limite de saturation, on doit effectuer l'opération en plusieurs fois, avec des séchages intermédiaires pour éliminer le solvant d'imprégnation, à une température de par exemple 90 à 250 °C, de préférence 100 à 180 °C. Ceci permet d'introduire de 0,01 à 30 %, de préférence de 1 à 20 %, et encore plus avantageusement de 2 à 12 % en poids sec de niobium et/ou de tantale.

Les composés de rhénium préférés sont l'heptoxyde de rhénium, le perrhénate d'ammonium et l'acide perrhénique. Le composé du rhénium peut être introduit sur le support par exemple par sublimation en phase vapeur ou par imprégnation en solution. On préfère généralement utiliser la méthode d'imprégnation à sec, décrite ci-dessus, où le composé du rhénium est mis en solution dans l'eau ou dans un solvant organique, par exemple un hydrocarbure, un alcool ou un éther. On règle la quantité de rhénium sur le support par le choix de la concentration de la solution d'imprégnation. Lorsque la quantité de rhénium que l'on désire imprégner est supérieure à celle que permet d'introduire une solution à sa limite de saturation, on doit effectuer l'opération en plusieurs fois, avec des séchages intermédiaires pour éliminer le solvant d'imprégnation, à une température de par exemple 90 à 250 °C, de préférence 100 à 180 °C. Ceci permet d'introduire de 0,01 à 20 %, de préférence de 0,1 à 15 %, et encore plus avantageusement de 0,5 à 8 % en poids sec de rhénium.

L'ordre d'introduction des composants : niobium et/ou tantale et rhénium, sur le support n'est pas critique. On préfère cependant introduire d'abord le niobium et/ou le tantale, puis le rhénium. Après chaque étape d'imprégnation, on effectue un séchage à une température de par exemple 90 à 250 °C, de préférence 100 à 180 °C, puis une calcination à une température de par exemple 250 à 700 °C, et de préférence 300 à 600 °C.

Le catalyseur peut avantageusement contenir, en plus du niobium et/ou du tantale et du rhénium, au moins un composé choisi dans le groupe formé par les composés alcalins et les composés alcalino-terreux, qui peuvent être de préférence ajoutés au catalyseur obtenu à la suite des étapes précédemment décrites. Ces composés peuvent être présents en proportions variables, généralement de 0,01 à 20 % et de préférence de 0,05 à 10 % en poids, et ils peuvent être introduits à partir de précurseurs, et par des méthodes, bien connus de l'homme de l'art.

L'activation de la composition catalytique obtenue à la suite des étapes précédentes s'effectue par chauffage entre 400 et 1000 °C, de préférence entre 500 et 900 °C. Ce chauffage se fait sous une atmosphère de gaz non réducteur, par exemple : oxygène, azote ou argon, oxygène dilué par de l'azote, de préférence sous air, sous conditions statiques ou dynamiques, un léger courant gazeux étant cependant préférable. Le taux d'humidité du courant gazeux est de préférence maintenu inférieur à 200 ppm (parties par million). On peut cependant effectuer le chauffage en atmosphère constituée par les gaz de combustion du méthane ou d'un gaz naturel en présence d'un excès d'air. La durée de ce traitement d'activation est par exemple de 10 minutes à 5 heures ou davantage, après quoi le catalyseur actif ainsi obtenu est refroidi sous atmosphère de préférence anhydre. On peut avantageusement procéder à une purge à l'azote, si nécessaire, avant mise en contact avec la charge hydrocarbonée.

Les oléfines susceptibles de réagir en métathèse en présence du catalyseur à base de rhénium supporté précédemment décrit peuvent être des oléfines linéaires répondant à la formule générale : R₁R₂C=CR₃R₄, où R₁, R₂, R₃ et R₄, identiques ou différents, sont l'hydrogène ou un radical hydrocarbyle de 1 à 20 atomes de carbone. Les oléfines peuvent aussi être de structure cyclique, le cycle comportant de 3 à 20 atomes de carbone. On peut soit faire réagir une oléfine sur elle-même, soit faire réagir plusieurs oléfines entre elles en mélange. Un exemple d'application est la production de propylène par réaction de l'éthylène avec les butènes-2, ou la réaction inverse de transformation du propylène en un mélange d'éthylène et de butènes-2.

La réaction de métathèse est réalisée de préférence en l'absence de solvant. Cependant, la présence d'un solvant tel qu'un hydrocarbure, ou un hydrocarbure halogéné, aliphatique, cyclanique ou aromatique, n'est pas néfaste.

La réaction peut être effectuée en batch dans un réacteur agité, ou en continu, en faisant passer le ou les réactifs à travers un lit fixe, un lit mobile ou un lit fluidisé, du catalyseur.

La réaction de métathèse est réalisée en phase gazeuse ou en phase liquide. On préfère généralement opérer en phase liquide, en l'absence d'oxygène et d'humidité. Les réactifs et solvants sont utilement traités en ce sens au préalable.

La pression à laquelle la réaction est effectuée n'est pas critique. Cependant, pour une opération en phase liquide, il est nécessaire de maintenir une pression au moins égale à la tension de vapeur du mélange réactionnel à la température de la réaction. On opère le plus souvent à une température comprise entre 0 et 200 °C, de préférence entre 20 et 150 °C.

Les exemples suivants illustrent l'invention sans en limiter la portée.

### EXEMPLE 1

### Préparation du catalyseur :

On calcine à 300 °C sous air 8,55 g d'une alumine gamma cubique ayant une surface spécifique de 184 m²/g et un volume poreux de 0,67 ml/g. Après refroidissement à température ambiante, on imprègne cette alumine avec une solution de 2,51 g de pentaéthoxyniobium dans 4 ml d'éthanol anhydre. Après 30 minutes de contact à température ambiante, on sèche le solide obtenu dans une étuve à 120 °C pendant une nuit. On le calcine ensuite sous un courant d'air d'environ 20 l/h à une température de 500 °C pendant 2 heures. On prépare une solution pour l'imprégnation du rhénium en diluant 0,23 ml d'une solution aqueuse concentrée d'acide perrhénique contenant 54,08 % en poids de rhénium (masse spécifique : 2,417 g/ml) dans 5 ml d'eau. Cette solution est imprégnée sur le solide précédemment calciné. Après 30 minutes de contact à température ambiante, on sèche le solide obtenu dans une étuve à 120 °C pendant une nuit. On le calcine ensuite sous un courant d'air (environ 20 l/h) séché par passage à travers un lit de tamis moléculaire, à une température de 550 °C pendant 2 heures. Pendant la période ultérieure de refroidissement, on substitue au courant d'air un courant d'azote sec. On obtient ainsi 10 g de catalyseur activé qui est conservé en atmosphère sèche et inerte avant utilisation. Sa teneur en oxyde de niobium est de 10,5 % en poids et sa teneur en rhénium métal est de 3 % en poids.

### Utilisation en métathèse :

Dans un réacteur constitué par un tube en acier inoxydable muni d'une double enveloppe avec circulation d'eau permettant la régulation de la température, on charge à l'abri de l'air et de l'humidité les 10 g de catalyseur préparés ci-dessus. Du propylène liquide est injecté au moyen d'une pompe par le bas du réacteur, avec un débit de 43,8 g/h. La température est réglée à 35 °C et la pression est maintenue à 3,5 MPa au moyen d'un régulateur placé en aval du réacteur. Dans ces conditions, la conversion du propylène à la sortie du réacteur est de 26,7 %, en un mélange équimolaire d'éthylène et de butènes-2.

### EXEMPLE 2

### Préparation du catalyseur :

Une solution aqueuse d'acide oxalique est préparée par dissolution de 46,8 g d'acide dans 331,4 g d'eau distillée. On prépare un gel d'oxyde de niobium hydraté par hydrolyse de pentachlorure de niobium dans une solution aqueuse d'ammoniaque, suivie de cinq lavages avec de l'eau distillée. On prélève 46,9 g de ce gel hydraté et on le dissout dans la solution aqueuse d'acide oxalique préparée ci-dessus. On calcine à 300 °C sous air 70 g d'une alumine gamma cubique ayant une surface spécifique de 184 m²/g et un volume poreux de 0,67 ml/g. Après refroidissement à température ambiante, on imprègne cette alumine avec 42 ml de la solution oxalique du gel d'oxyde de niobium préparée ci-dessus. Après 30 minutes de contact à température ambiante on sèche le solide obtenu dans une étuve à 120 °C. Cette opération d'imprégnation suivie de séchage est répétée neuf fois. Après quoi, le solide obtenu est calciné sous un courant d'air d'environ 20 l/h à une température de 500 °C pendant 2 heures. Sa teneur en oxyde de niobium est de 9,6 % en poids. On prélève 9,61 g de ce solide que l'on imprègne avec une solution préparée en diluant 0,23 ml d'une solution aqueuse concentrée d'acide perrhénique contenant 54,08 % en poids de rhénium (masse spécifique : 2,417 g/ml) dans 5 ml d'eau. Après 30 minutes de contact à température ambiante, on sèche le solide obtenu dans une étuve à 120 °C pendant une nuit. On le calcine ensuite sous un courant d'air (environ 20 l/h) séché par passage à travers un lit de tamis moléculaire, à une température de 550 °C pendant 2 heures. Pendant la période ultérieure de refroidissement, on substitue au courant d'air un courant d'azote sec. On obtient ainsi 10 g de catalyseur activé qui est conservé en atmosphère sèche et inerte avant utilisation. Sa teneur en rhénium métal est de 3 % en poids.

### Utilisation en métathèse :

Dans le même appareillage que celui décrit dans l'exemple 1, on charge les 10 g de catalyseur préparés ci-dessus. Du propylène liquide est injecté avec un débit de 43,8 g/h, à une température de 35 °C et sous une pression de 3,5 MPa. Dans ces conditions, la conversion du propylène est de 23,8 %.

### EXEMPLE 3(Comparatif)

### Préparation du catalyseur :

Un nouveau lot de catalyseur est préparé comme dans l'exemple 1, à cette différence près qu'on utilise 9,61 g d'alumine et que l'on omet l'étape d'imprégnation par le composé de niobium. L'étape d'imprégnation du rhénium ainsi que la phase de séchage et de calcination finale sont identiques à celles décrites dans l'exemple 1. On obtient ainsi 10 g de catalyseur activé qui est conservé en atmosphère sèche et inerte avant utilisation. Sa teneur en rhénium métal est de 3 % en poids.

### Utilisation en métathèse :

Dans le même appareillage que celui décrit dans l'exemple 1, on charge les 10 g de catalyseur préparés ci-dessus. Du propylène liquide est injecté avec un débit de 43,8 g/h, à une température de 35 °C et sous une pression de 3,5 MPa. Dans ces conditions, la conversion du propylène est de 7,1 %.

Cet exemple montre la moins bonne activité d'un catalyseur de l'art antérieur.

### EXEMPLE 4(Comparatif)

### Préparation du catalyseur :

Un nouveau lot de catalyseur est préparé comme dans l'exemple 1, à cette différence près qu'on utilise 8,96 g d'alumine et que l'on omet l'étape d'imprégnation par le composé de niobium. Dans l'étape d'imprégnation du rhénium, on utilise une solution obtenue en diluant 0,61 ml de la solution concentrée d'acide perrhénique dans 5 ml d'eau. Après séchage dans une étuve à 120 °C pendant une nuit, on calcine ensuite le catalyseur sous un courant de gaz de combustion de méthane à une température de 750 °C pendant 1 heure. Pendant la période ultérieure de refroidissement, on substitue au courant d'air un courant d'azote sec. On obtient ainsi 10 g de catalyseur activé qui est conservé en atmosphère sèche et inerte avant utilisation. Sa teneur en rhénium métal est de 8 % en poids.

### Utilisation en métathèse :

Dans le même appareillage que celui décrit dans l'exemple 1, on charge les 10 g de catalyseur préparés ci-dessus. Du propylène liquide est injecté avec un débit de 43,8 g/h, à une température de 35 °C et sous une pression de 3,5 MPa. Dans ces conditions, la conversion du propylène est de 22,6 %.

Cet exemple, comparé aux exemples 1 et 2, montre qu'un catalyseur de l'art antérieur contenant 8 % de rhénium est moins actif qu'un catalyseur selon l'invention modifié avec de l'oxyde de niobium et contenant seulement 3 % de rhénium.

### EXEMPLE 5

### Préparation du catalyseur :

On calcine à 300 °C sous air 7,85 g d'une alumine gamma cubique ayant une surface spécifique de 184 m²/g et un volume poreux de 0,67 ml/g. Après refroidissement à température ambiante, on imprègne cette alumine avec une solution de 3,23 g de pentaéthoxytantale dans 5 ml d'éthanol anhydre. Après 30 minutes de contact à température ambiante, on sèche le solide obtenu dans une étuve à 120 °C pendant une nuit. On le calcine ensuite sous un courant d'air d'environ 20 l/h à une température de 500 °C pendant 2 heures. On prépare une solution pour l'imprégnation du rhénium en diluant 0,23 ml d'une solution aqueuse concentrée d'acide perrhénique contenant 54,08 % en poids de rhénium (masse spécifique : 2,417 g/ml) dans 5 ml d'eau. Cette solution est imprégnée sur le solide précédemment calciné. Après 30 minutes de contact à température ambiante, on sèche le solide obtenu dans une étuve à 120 °C pendant une nuit. On le calcine ensuite sous un courant d'air (environ 20 l/h) séché par passage à travers un lit de tamis moléculaire, à une température de 550 °C pendant 2 heures. Pendant la période ultérieure de refroidissement, on substitue au courant d'air un courant d'azote sec. On obtient ainsi 10 g de catalyseur activé qui est conservé en atmosphère sèche et inerte avant utilisation. Sa teneur en oxyde de tantale est de 18,3 % en poids et sa teneur en rhénium métal est de 3 % en poids.

### Utilisation en métathèse :

Dans le même appareillage que celui décrit dans l'exemple 1, on charge les 10 g de catalyseur préparés ci-dessus. Du propylène liquide est injecté avec un débit de 43,8 g/h, à une température de 35 °C et sous une pression de 3,5 MPa. Dans ces conditions, la conversion du propylène est de 12,9 %.

## Revendications

1. Procédé de métathèse des oléfines, mettant en oeuvre un catalyseur au rhénium, caractérisé en ce que le dit catalyseur comprend un support contenant au moins 75 % en poids d'alumine, 0,01 à 20 % en poids de rhénium sous forme d'oxyde et 0,01 à 30 % en poids d'au moins un oxyde d'un métal choisi dans le groupe formé par le niobium et le tantale.

2. Procédé selon la revendication 1, caractérisé en ce que le catalyseur est obtenu à partir d'une composition catalytique formée à partir d'au moins un support poreux contenant au moins 75 % en poids d'alumine, un composé d'au moins un métal choisi dans le groupe constitué par le niobium et le tantale, et d'au moins un composé du rhénium, ladite composition étant activée en atmosphère non réductrice.

3. Procédé selon l'une des revendications 1 ou 2, caractérisé en ce qu'est également présent au moins un élément choisi dans le groupe formé par les alcalins et les alcalino-terreux.

4. Procédé selon la revendication 2, dans lequel le composé du niobium est choisi dans le groupe formé par les halogénures, les oxyhalogénures, les alkoxydes, les oxydes hydratés complexés par de l'acide oxalique, les oxydes hydratés complexés par de l'acide tartrique et les sels d'ammonium desdits acides.

5. Procédé selon la revendication 2, dans lequel le composé du tantale est choisi dans le groupe formé par les halogénures, les oxyhalogénures, les alkoxydes, les oxydes hydratés complexés par de l'acide oxalique, les oxydes hydratés complexés par de l'acide tartrique et les sels d'ammonium desdits acides.

6. Procédé selon l'une des revendications précédentes, dans lequel le composé du rhénium appartient au groupe constitué par l'heptoxyde de rhénium, le perrhénate d'ammonium et l'acide perrhénique.

7. Procédé selon l'une des revendications précédentes, dans lequel le support poreux est l'alumine de porosité au moins égale à 0,1 ml/g et de surface au moins égale à 10 m²/g.

8. Procédé selon l'une des revendications précédentes, dans lequel le catalyseur est préparé par introduction sur le support du composé du métal choisi dans le groupe formé par le niobium et le tantale, puis introduction du composé du rhénium.

9. Procédé selon l'une des revendications précédentes, dans lequel la réaction de métathèse est effectuée en phase liquide à une température comprise entre 0 et 200 °C, et sous une pression au moins égale à la tension de vapeur du milieu réactionnel à la température de réaction.

10. Procédé selon l'une des revendications précédentes, dans lequel les oléfines de la réaction de métathèse sont choisies dans le groupe formé par les oléfines linéaires et les oléfines cycliques.

## Claims

1. A process for the metathesis of olefins, employing a rhenium catalyst, characterised in that said catalyst comprises a support containing at least 75% by weight of alumina, 0.01 to 20% by weight of rhenium in oxide form and 0.01 to 30% by weight of at least one oxide of a metal selected from the group consisting of niobium and tantalum.

2. A process according to Claim 1, characterised in that the catalyst is obtained from a catalytic composition formed from at least one porous support containing at least 75% by weight of alumina, a compound of at least one metal selected from the group consisting of niobium and tantalum, and at least one rhenium compound, said composition being activated in a non-reducing atmosphere.

3. A process according to one of Claims 1 or 2, characterised in that at least one element selected from the group consisting of alkali metals and alkaline earth metals is also present.

4. A process according to Claim 2, in which the niobium compound is selected from the group consisting of halides, oxyhalides, alkoxides, hydrated oxides complexed by oxalic acid, hydrated oxides complexed by tartaric acid and ammonium salts of said acids.

5. A process according to Claim 2, in which the tantalum compound is selected from the group consisting of halides, oxyhalides, alkoxides, hydrated oxides complexed by oxalic acid, hydrated oxides complexed by tartaric acid and ammonium salts of said acids.

6. A process according to one of the preceding claims, in which the rhenium compound belongs to the group consisting of rhenium heptoxide, ammonium perrhenate and perrhenic acid.

7. A process according to one of the preceding claims, in which the porous support is alumina with a porosity which is at least equal to 0.1 ml/g and with a surface area which is at least equal to 10 m²/g.

8. A process according to one of the preceding claims, in which the catalyst is prepared by introducing onto the support the compound metal selected from the group consisting of niobium and tantalum, and then by introducing the rhenium compound.

9. A process according to one of the preceding claims, in which the metathesis reaction is carried out in liquid phase at a temperature of between 0 and 200 °C, and at a pressure which is at least equal to the vapour pressure of the reaction medium at the reaction temperature.

10. A process according to one of the preceding claims, in which the olefins of the metathesis reaction are selected from the group consisting of linear olefins and cyclic olefins.

## Patentansprüche

1. Verfahren zur Methathese von Olefinen unter Verwendung eines Rheniumkatalysators, dadurch gekennzeichnet, daß dieser Katalysator einen Träger, der wenigstens 75 Gew.% Aluminiumoxid enthält, 0,01 bis 20 Gew.% Rhenium in Form von Oxid und 0,01 bis 30 Gew.% wenigstens eines Oxids eines Metalls enthält, das aus der durch Niob und Tantal gebildeten Gruppe ausgewählt ist.

2. Verfahren nach Anspruch 1, dadurch gekennzeichnet, daß der Katalysator aus einer katalytischen Zusammensetzung erhalten wird, die aus wenigstens einem porösen Träger, der mindestens 75 Gew.% Aluminiumoxid enthält, einer Verbindung wenigstens eines aus der durch Niob und Tantal gebildeten Gruppe ausgewählten Metalls und wenigstens einer Rheniumverbindung gebildet ist, wobei die Zusammensetzung in nicht reduzierender Atmosphäre aktiviert wird.

3. Verfahren nach einem der Ansprüche 1 oder 2, dadurch gekennzeichnet, daß ebenfalls wenigstens ein aus der durch die Alkalien und Erdalkalien gebildeten Gruppe ausgewähltes Element vorhanden ist.

4. Verfahren nach Anspruch 2, bei dem die Niobverbindung aus der Gruppe ausgewählt ist, die durch die Halogenide, die Oxihalogenide, die Alkoxide, die mit Oxalsäure komplexierten wasserhaltigen Oxide, die mit Weinsäure komplexierten wasserhaltigen Oxide und die Ammoniumsalze dieser Säuren gebildet ist.

5. Verfahren nach Anspruch 2, bei dem die Tantalverbindung aus der Gruppe ausgewählt ist, die durch die Halogenide, die Oxihalogenide, die Alkoxide, die mit Oxalsäure komplexierten wasserhaltigen Oxide, die mit Weinsäure komplexierten wasserhaltigen Oxide und die Ammoniumsalze dieser Säuren gebildet ist.

6. Verfahren nach einem der vorhergehenden Ansprüche, bei dem die Rheniumverbindung zu der Gruppe gehört, die durch Rheniumheptoxid, Ammoniumperrhenat und Perrheniumsäure gebildet ist.

7. Verfahren nach einem der vorhergehenden Ansprüche, bei dem der poröse Träger Aluminiumoxid mit einer Porosität von mindestens gleich 0,1 ml/g und einer Oberfläche von mindestens gleich 10 m²/g ist.

8. Verfahren nach einem der vorhergehenden Ansprüche, bei dem der Katalysator durch Einführen der Verbindung des aus der durch Niob und Tantal gebildeten Gruppe ausgewählten Metalls und dann Einführen der Rheniumverbindung auf den Träger hergestellt wird.

9. Verfahren nach einem der vorhergehenden Ansprüche, bei dem die Methathesereaktion in flüssiger Phase bei einer Temperatur von zwischen 0 und 200°C und unter einem Druck mindestens gleich dem Dampfdruck des Reaktionsmediums bei der Reaktionstemperatur durchgeführt wird.

10. Verfahren nach einem der vorhergehenden Ansprüche, bei dem die Olefine der Methathesereaktion ausgewählt sind aus der durch die linearen und die zyklischen Olefine gebildeten Gruppe.
